# EUROPEAN PATENT SPECIFICATION

(11) **EP 2 737 885 B1**
(45) Date of publication and mention of the grant of the patent: **21.11.2018**
(21) Application number: 12818281.3
(22) Date of filing: 19.07.2012
(51) Int. Cl.: A61F 13/15, A61F 13/49

(54) **ABSORBENT ARTICLE PRODUCTION EQUIPMENT**
VORRICHTUNG ZUR HERSTELLUNG EINES SAUGFÄHIGEN ARTIKELS
ÉQUIPEMENT DE FABRICATION D'ARTICLES ABSORBANTS

(30) Priority: 25.07.2011 JP 2011162129
(43) Date of publication of application: 04.06.2014
(73) Proprietor: Unicharm Corporation, Ehime 799-0111 (JP)
(72) Inventor: YAMAMOTO, Hiroki, Kanonji-shi Kagawa 769-1602 (JP)
(74) Representative: Staeger & Sperling Partnerschaftsgesellschaft mbB
(86) International application number: PCT/JP2012/068287
(87) International publication number: WO 2013/015183

(56) References cited:
- EP-A1- 1 013 588
- WO-A1-99/18019
- JP-A- 4 087 929
- JP-A- 2001 519 302
- JP-A- 2007 516 908
- JP-A- 2009 028 545
- US-A- 4 325 475
- US-A- 5 127 209
- US-A1- 2004 068 967

## Description

### Technical Field

The present invention relates to a device for manufacturing an absorbent article having a counting mechanism for counting the absorbent articles supplied in continuation.

### Background Art

Patent Literature 1 describes a counting machine for counting absorbent articles, such as a disposable worn article. The counting machine described in Patent Literature 1 includes an endless belt for conveying the absorbent articles in a predetermined direction, and a plurality of guide plates installed vertically in the entire outer circumference surface of the endless belt, and is configured to convey the absorbent articles by storing the absorbent articles between the guide plates.

### Prior Art Document

### Patent Document

Patent Document 1: Japanese Patent Application Publication 63-277125 (Fig. 1 and others).

### Summary of Invention

However, as described above, the use of the aforementioned counting machine had the following problem.

The absorbent articles supplied in continuation are fed one by one towards the space between the guide plates of the counting machine. At this time, as shown in Fig. 1 of Patent Literature 1, the absorbent articles are fed from the tip, which is the protruding tip side of the guide plate, towards the base end, which is the connecting side with the endless belt. That is, the absorbent articles are fed towards the endless belt positioned between the guide plates. Therefore, for example, when an absorbent article is fed with force, the absorbent article may collide with the endless belt, and the position of the absorbent article between the guide plates might shift, and the absorbent article might be thrown out from between the guide plates towards the outside. Further prior art in this technical field is disclosed in documents WO99/18019 A1, US 5,127,209 A, EP 1 013 588 A1, US 2004/068967 A1 and US 4,325,475 A.

Therefore, the present invention has been achieved in view of such a situation, and an object thereof is to provide a device for manufacturing an absorbent article by which absorbent articles can be counted by storing the absorbent articles in a stable state between guide members.

According to the present disclosure, a device for manufacturing an absorbent article (device 100 for manufacturing an absorbent article) comprising: a forming mechanism (forming mechanism 65) of forming a plurality of absorbent articles including a front waistline region (front waistline region 10), a back waistline region (back waistline region 20), and a crotch region (crotch region 30) positioned between the front waistline region and the back waistline region, by arranging an absorber on a web (web 7) in which the components configuring the absorbent articles are in continuity, followed by folding the web along a conveyance direction, and then cutting the web along the side surfaces in a widthwise direction of the absorbent articles positioned at a side of the front waistline region and at a side of the back waistline region; and a conveyance mechanism (conveyance mechanism 75) of continuously conveying the absorbent articles formed by the forming mechanism; and a counting mechanism (counting mechanism 90) of counting the absorbent articles supplied in continuation from the conveyance mechanism. The counting mechanism includes a plurality of guide members (guide members 92) configured to retain the absorbent articles, a movement member (movement member 91) configured to move the guide members in a direction along a horizontal direction, and support members (support members 93) arranged on a lower side of the guide members in a vertical direction, and is configured to move the absorbent articles supplied from the conveyance mechanism while being sandwiched by the guide members, and then count the absorbent articles, and the guide members protrude out from the outer circumference surface of the movement member along the horizontal direction, and are arranged adjacent to each other along the horizontal direction, and opposing surfaces opposing each other in the guide members are the surfaces that sandwich the absorbent articles, and are arranged along the vertical direction, and the conveyance mechanism is configured to feed the absorbent articles along the opposing surfaces, from the upper side of the guide members in the vertical direction towards the space between the guide members. The support members are configured from a sponge or a rubber, or a sponge or a rubber is provided on an upper surface of the support members.

In the conveyance mechanism of the device for manufacturing an absorbent article according to the present invention, the absorbent articles are fed from an upper side of the guide member towards a lower side. Support members are arranged at the lower side of the guide member, and when the absorbent articles are fed with force, the absorbent articles may collide with the support members. However, because the absorbent articles are fed by moving in a direction along a vertical direction, even when the absorbent articles collide with the support members and bounce, the absorbent articles stay between the guide members due to the force of gravity. Thus, the absorbent articles can be stored in a stable state.

### Brief Description of Drawings

[Fig. 1] Fig. 1 is a perspective view showing an absorbent article according to the present embodiment.
[Fig. 2] Fig. 2 is a diagram for explaining a part of a process of manufacturing the absorbent article according to the present embodiment.
[Fig. 3] Fig. 3 is a diagram schematically showing a part of a device for manufacturing an absorbent article according to a first embodiment.
[Fig. 4] Fig. 4 is a diagram schematically showing a part of a counting mechanism configuring the device for manufacturing an absorbent article according to the first embodiment.
[Fig. 5] Fig. 5 is a diagram schematically showing a part of a device for manufacturing an absorbent article according to a second embodiment.

### Description of Embodiments

[Fig. 6] Fig. 6 is a diagram schematically showing a part of a counting mechanism configuring the device for manufacturing an absorbent article according to the second embodiment.
[Fig. 7] Fig. 7 is a diagram schematically showing a part of the counting mechanism configuring the device for manufacturing an absorbent article according to the second embodiment.
[Fig. 8] Fig. 8 is a diagram schematically showing a part of the counting mechanism configuring the device for manufacturing an absorbent article according to the second embodiment.

### Description of Embodiments

Next, a device for manufacturing an absorbent article according to a first embodiment and a second embodiment is explained with reference to drawings. Specifically, (1) Configuration of absorbent article, (2) Device for manufacturing absorbent article according to first embodiment, (3) Configuration of counting mechanism, and (4) Device for manufacturing absorbent article according to second embodiment are explained.

In the following description of the drawings, the same or similar reference numerals are used to designate the same or similar parts. It will be appreciated that the drawings are schematically shown and the ratio and the like of each dimension are different from the real ones. Therefore, a specific dimension should be determined in view of the following description. Moreover, among the drawings, the respective dimensional relations or ratios may naturally differ.

### (1) Configuration of absorbent article

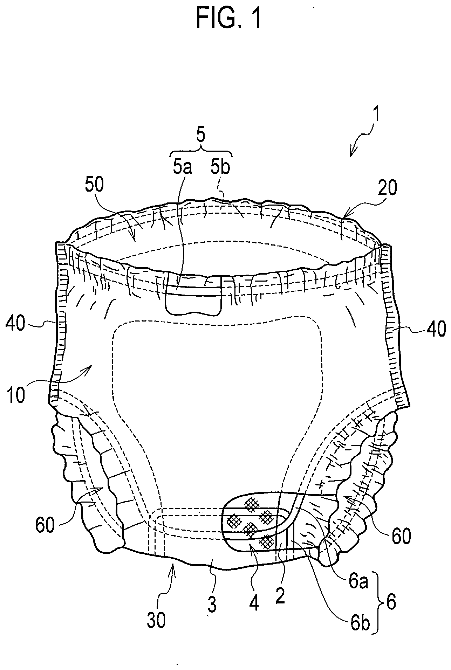

First of all, a configuration of an absorbent article according to the present embodiment is explained with reference to drawings. Fig. 1 is a perspective view showing the absorbent article according to the present embodiment. In the present embodiment, an absorbent article 1 is a disposable diaper for adults.

As shown in Fig. 1, roughly, the absorbent article 1 is configured by: a liquid-permeable topsheet 2 that is in contact with the skin of the wearing subject (hereinafter, the wearer); a backsheet 3 provided on the outer side from the topsheet 2; and an absorber 4 that is provided between the topsheet 2 and the backsheet 3 and absorbs the bodily waste from the wearer.

Note that a liquid-impermeable water-resistive sheet (not shown in the figure) is provided between the backsheet 3 and the absorber 4. In other words, the absorber 4 is provided between the topsheet 2 and the water-resistive sheet.

A nonwoven fabric or an aperture plastic film, for example, is used in the topsheet 2. A nonwoven fabric is used in the backsheet 3. Ground pulp, or a mixture of ground pulp and high absorbent polymer grains, for example, is used in the absorber 4. A plastic film, a nonwoven fabric, or a combined sheet of a plastic fiim and a nonwoven fabric, for example, is used in the water-resistive sheet.

The absorbent article 1 has a front waistline region 10 corresponding to the front waistline of the wearer, a back waistline region 20 corresponding to the back waistline of the wearer, and a crotch region 30 corresponding to the crotch of the wearer.

The front waistline region 10 and the back waistline region 20 are formed as one part by a joining unit 40. Waist gathers 5 made from thread-shaped rubber, for example, having elasticity are provided on the circumference of the front waistline region 10 and the back waistline region 20. The waist gathers 5 are configured from an anterior waist gathers 5a positioned in the front waistline region 10 and a posterior waist gathers 5b positioned in the back waistline region 20. A waistline opening region 50 is formed between the anterior waist gathers 5a and the posterior waist gathers 5b.

The crotch region 30 is provided between the front waistline region 10 and the back waistline region 20. Leg gathers 6 made from thread-shaped or band-shaped rubber having elasticity are provided on both sides of the crotch region 30. The leg gathers 6 are configured from an anterior leg gathers 6a positioned towards the front waistline region 10 and a posterior leg gathers 6b positioned towards the back waistline region 20. A leg-hole opening region 60 is formed between the anterior leg gathers 6a and the posterior leg gathers 6b, and on both sides of the crotch region 30.

### (2) Device for manufacturing absorbent article according to first embodiment

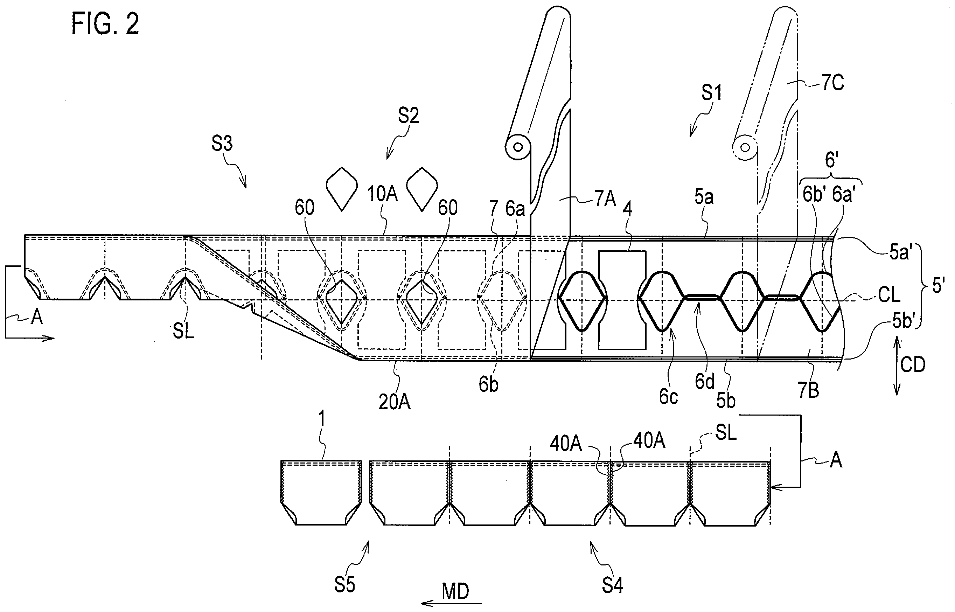

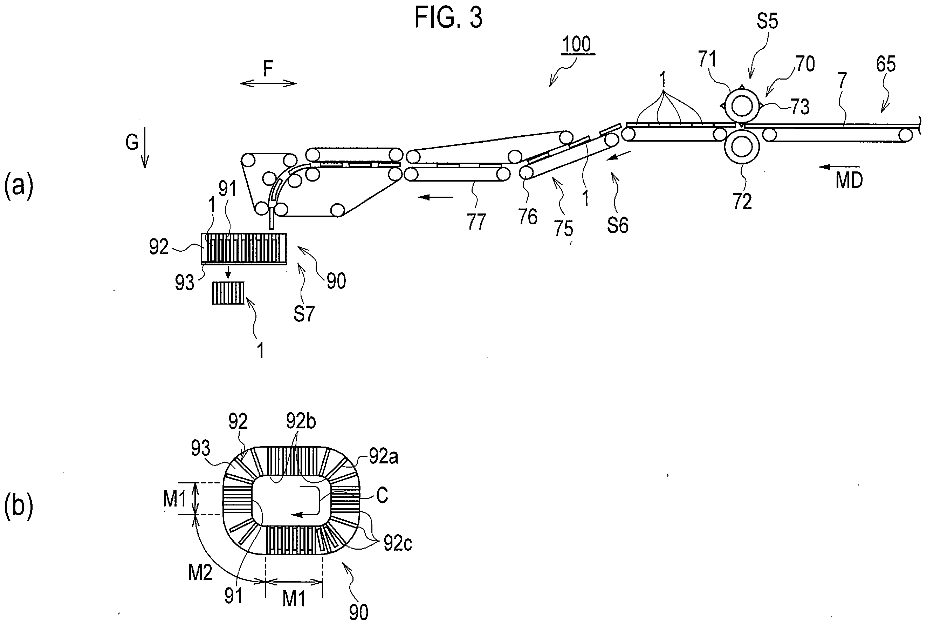

Next, a configuration of a device 100 for manufacturing an absorbent article according to the first embodiment is explained along a process of manufacturing the absorbent article. Fig. 2 is a diagram for explaining the process of manufacturing the absorbent article by the device for manufacturing the absorbent article. Fig. 2 is a schematic view when the process of manufacturing the absorbent article is visually recognized from the top, and is a diagram for explaining a component loading step, a leg hole forming step, a folding step, a joining step, and a cutting step, which constitute a forming step of the absorbent article. Fig. 3 (a) is a schematic view when the device for manufacturing the absorbent article is visually recognized from the side, and is a diagram for explaining a part of a forming mechanism 65 by which the forming step of the device for manufacturing the absorbent article is performed, a conveyance mechanism, and a counting mechanism.

The method for manufacturing the absorbent article includes at least a forming step, a conveyance step, and a counting step. The forming step based on the forming mechanism 65 includes the component loading step, the leg hole forming step, the folding step, the joining step, and the cutting step. A step of conveying a liquid-permeable first web 7A configuring the topsheet 2, a liquid-impermeable second web 7B configuring the backsheet 3, and a third web 7C made from materials such as the same material as that of the second web 7B and configuring the backsheet 3 in a conveyance direction MD by a conveyor (for example, a belt conveyor), which is not shown in the figure, is included in the forming step.

### (2-1) Component loading step

In a component loading step S1, the components configuring the absorbent article 1, such as the elastic members, the third web 7C, the water-resistive sheet (not shown in the figure), and the absorber 4 are loaded on the second web 7B.

### (2-2) Leg hole forming step

In a leg hole forming step S2, after the component loading step S1, the leg-hole opening region 60 (the so-called leg hole) is formed on the second web 7B and the first web 7A (hereinafter, web 7) between which the components are held, by cutting out both sides of the crotch region 30.

### (2-3) Folding step

In a folding step S3, after the leg hole forming step S2, a web 7 is folded into two through the center of a direction CD crossing the web 7 and along a central line CL facing the conveyance direction MD. In other words, a side edge 10A of the web 7 corresponding to the front waistline region 10, and a side edge 20A of the web 7 corresponding to the back waistline region 20 overlap in a matching state.

### (2-4) Joining step

In a joining step S4, after the folding step S3, a predetermined region 40A corresponding to the joining unit 40 of the absorbent article 1 is joined with an ultrasonic treatment and a heat treatment. Note that the predetermined region 40A shows both sides of the conveyance direction MD of the virtual line SL showing the estimated cutting position extending in the crossing direction CD.

### (2-5) Cutting step

In a cutting step S5, after the joining step S4, the web 7 with which the predetermined region 40A is joined is cut along the virtual line SL by a cutting device 70 shown in Fig. 3(a). The absorbent article 1 is thus formed. The cutting device 70 has a cutter roller 71 and an anvil roller 72 arranged opposite to each other. A cutting blade 73 configured to cut the web 7 is provided on the outer circumference surface of the cutter roller 71. The web 7 is cut by the cutting blade 73 at a fixed interval when the web 7 passes through between the cutter roller 71 and the anvil roller 72.

### (2-6) Conveyance step

In a conveyance step S6, the absorbent article 1 that is cut in the cutting step S5 is conveyed towards a counting mechanism 90 by a conveyance mechanism 75. The conveyance mechanism 75 is configured by an endless belt 77 suspended on a plurality of rollers 76.

In the conveyance mechanism 75, if necessary, the gap between the conveyed absorbent articles 1 can be adjusted. Specifically, for example, by switching to a conveyance path having a different conveyance velocity, the gap between the absorbent articles 1 can be adjusted.

### (2-7) Counting step

In a counting step S7, the absorbent articles 1 conveyed by the conveyance mechanism 75 are counted by the counting mechanism 90. The counting mechanism 90 includes an endless belt 91 as a movement member configured to move in a fixed direction, a plurality of guide members 92 protruding out along a horizontal direction F from the outer circumference surface of the endless belt 91, and support members 93 arranged at the lower side in a vertical direction G of the guide members 92. Fig. 3(b) is a diagram showing the state when the counting mechanism 90 shown in Fig. 3(a) is visually recognized from the upper side.

### (3) Configuration of counting mechanism

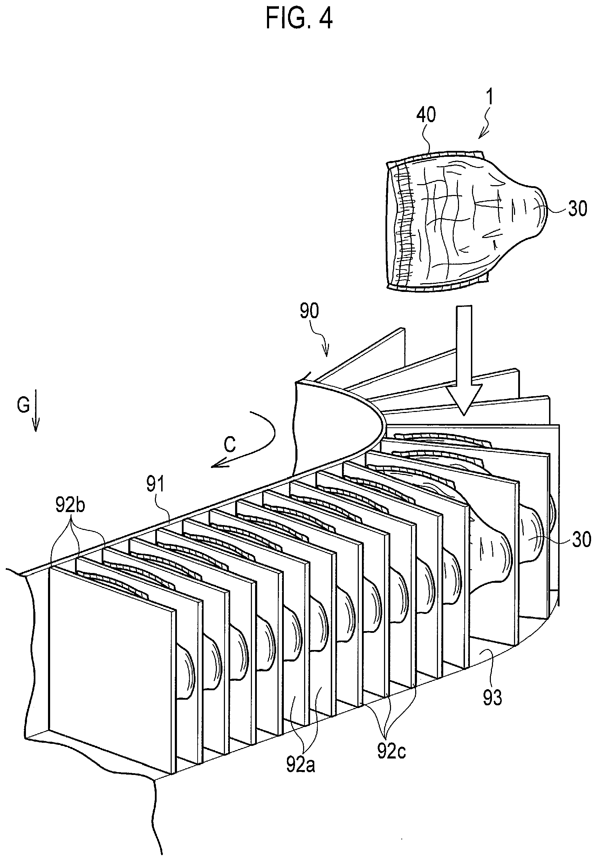

The counting mechanism 90 is configured to move the absorbent articles 1 supplied from the conveyance mechanism 75 while being sandwiched by the guide members 92, and then count the absorbent articles 1. Fig. 4 is a perspective view that schematically shows a part of the counting mechanism.

The endless belt 91 is moved in a direction C shown in Fig. 3(b) and Fig. 4, by a drive member (for example, a motor), which is not shown in the figure. The guide members 92 protrude out from the outer circumference surface of the movement member along the horizontal direction F, and are arranged adjacent to each other along the horizontal direction F. As a result of the movement of the endless belt 91 in the direction C, the guide members 92 move in the direction C.

The guide members 92 are in the shape of thin plates, and opposing surfaces 92a that are adjacent to each other in a plurality of guide members are arranged along the vertical direction G. The guide members 92 are arranged so as to enclose the opposing surfaces 92a, and include a pair of side surfaces arranged along the vertical direction. The pair of side surfaces of the guide members 92 have a connecting side surface 92b, which is a connecting unit connected with the endless belt 91, and a tip side surface 92c, which is the tip side in the direction of protrusion with respect to the endless belt 91.

The distance between the guide members in the connecting side surface 92b of the guide members 92 is constant because of being connected to the endless belt, and on the other hand, the distance between the guide members in the tip side surface 92c of the guide members is not constant because of not being connected to the endless belt, and the distance varies depending on the angle of movement of the endless belt.

Specifically, in a state as visually recognized from the top (the state shown in Fig. 3 (b)), the movement path of the endless belt 91 includes a linear first movement path M1 and an arc-shaped second movement path M2. In the linear first movement path M1, the distance between the guide members in the tip side surface 92c of the guide members 92 is constant. However, in the curved second movement path M2, the distance between the guide members in the tip side surface 92c of the guide members 92 is not constant, and is wider than the distance in the first movement path M1.

Thus, an interval between adjacent guide members varies depending on the position of the guide members. The conveyance mechanism feeds the absorbent articles to the guide members 92 of the second movement path M2 having a relatively wider interval. The guide members 92 convey the absorbent articles 1 fed in the second movement path M2 while sandwiching the absorbent articles between the guide members 92, and align the absorbent articles in the process of conveying in the first movement path M1.

The counting mechanism 90 has a pusher (not shown in the figure) that pushes the absorbent articles 1 conveyed in the aligned state in a lower direction. Thus, the absorbent articles are pushed out in every predetermined quantity. Though not shown in the figure, after the counting step S7, the absorbent articles pushed out in every predetermined quantity are stored in a box-shaped package and the like in every predetermined quantity of the absorbent articles.

The opposing surfaces 92a that face each other in the guide members 92 are the surfaces that sandwich the absorbent articles 1, and are arranged along the vertical direction G. The conveyance mechanism 75 feeds the absorbent articles 1 along the opposing surfaces 92a, from the upper side of the guide members 92 towards the space between the guide members 92. The fed absorbent articles 1 come in contact with the support members 93 between the guide members 92, and are stored between the guide members 92.

Thus, by feeding the absorbent articles 1 between the guide members 92 along the vertical direction G, the absorbent articles 1 can be stored in the counting mechanism 90 in a stable state. Specifically, for example, when the absorbent articles 1 are fed with force, the absorbent articles 1 may collide with the support members 93. However, because the absorbent articles 1 are fed with moving in a direction along the vertical direction G, even when the absorbent articles collide with the support members 93 and bounce, the absorbent articles stay between the guide members due to the force of gravity. Thus, the absorbent articles can be stored in a stable state.

Furthermore, the conveyance mechanism 75 feeds the absorbent articles 1 between the guide members 92 from the joining unit 40 side, which is the side surface in the widthwise direction W. The absorber 4 that is arranged in the crotch region 30 is not arranged in the joining unit 40 side of the absorbent article 1. Therefore, the impact occurring due to the collision with the support members 93 cannot be absorbed by the absorber 4, and as a result, the absorbent articles 1 bounce easily as compared to the case when the absorbent articles are fed from the bottom surface side to the space between the guide members 92. However, even when the absorbent articles are fed with force, the absorbent articles 1 stay in between the guide members 92 due to force of gravity, and the absorbent articles 1 can be stored in a stable state.

Additionally, because the absorbent articles are fed from the upper side of the guide members, a feeding position of the absorbent articles can be adjusted in a direction along the opposing surface of the guide members. Furthermore, the feeding position of the absorbent articles can be adjusted also in the vertical direction. Therefore, in cases where the arrangement of the counting mechanism and the conveyance mechanism in the device for manufacturing an absorbent article is changed, for example, the feeding position can be adjusted easily.

Furthermore, because the absorbent articles 1 fed from the conveyance mechanism come in contact with the support members 93, the absorbent articles 1 can be stored in a more secure and smooth manner by appropriately changing the material and configuration of the support members 93. Specifically, according to the invention, by providing an impact-buffer material, which buffers the impact, on the upper surface of the support members 93, or by configuring the support members 93 from a material that buffers the impact, such as sponge and rubber, the impact caused by the collision of the absorbent articles 1 is absorbed, and the absorbent articles can be stored in a stable manner. Furthermore, in order to enable more secure storage of the absorbent articles, the position of the support members can be configured to be adjusted by driving the support members.

Additionally, because the strength of the absorbent article is relatively low as the absorbent article is configured from a nonwoven fabric and an absorber, the absorbent article is prone to deformation in the conveyance process. However, by conveying the absorbent article while sandwiching between the guide members, the deformation of the absorbent article in the thickness direction is controlled, and the deformation and tilting of the entire absorbent article can be controlled.

### (4) Device for manufacturing absorbent article according to second embodiment

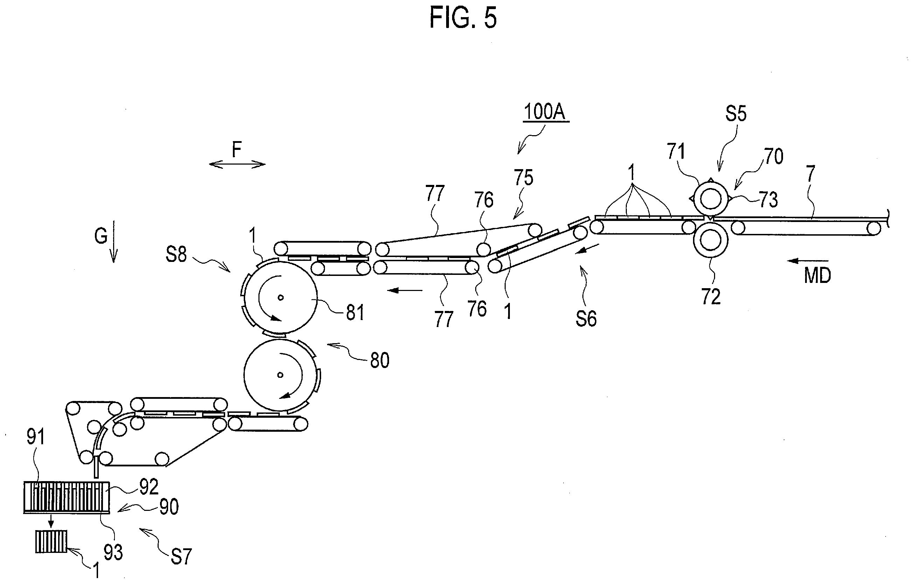

Next, a device 100A for manufacturing an absorbent article according to a second embodiment is explained based on Fig. 5 through Fig. 8. In the following explanation of the embodiment, only the configuration that is different from the first embodiment is explained, and the same signs are used for the configuration that is the same as the first embodiment while omitting the explanation.

The device 100A for manufacturing an absorbent article according to the second embodiment further includes a direction-changing mechanism 80 by which a direction-changing step for changing the direction of the absorbent articles formed by the forming mechanism 65 is performed.

The direction-changing mechanism includes a rotary drum 81 and a plurality of adsorption units (not shown in the figure) provided in the outer circumference surface of the rotary drum 81. The adsorption units are configured so as to absorb the absorbent articles 1, and rotate the absorbent articles 1 by 90 degrees with respect to the outer circumference surface of the rotary drum 81 while moving along the outer circumference surface of the rotary drum 81. The direction of the absorbent articles is thus changed.

Therefore, because the direction of the absorbent articles can be changed appropriately by the conveyance mechanism, the direction of the absorbent articles to be fed to the counting mechanism 90 can be changed.

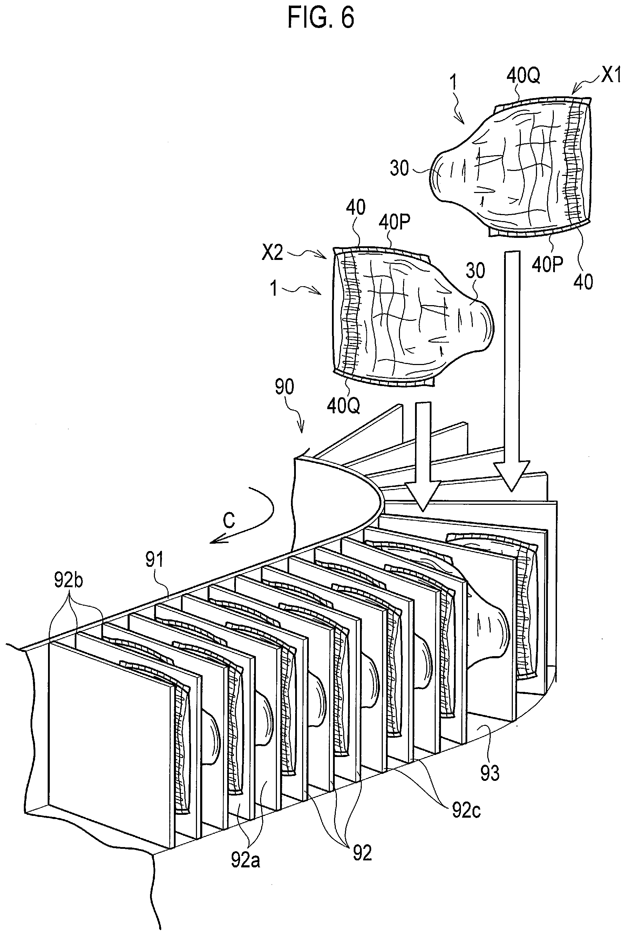

For example, by changing the direction of the adjacent absorbent articles 1 with the direction-changing mechanism 80 such that the direction of the absorbent articles changes mutually by 180 degrees, the conveyance mechanism 75 can alternately execute a first feeding form X1 for feeding the absorbent articles from one of the two side surfaces of the absorbent articles 1, that is, from a first side surface 40P side towards the space between the guide members, and a second feeding form X2 for feeding the absorbent articles from the other side of the two side surfaces of the absorbent articles 1, that is, from a second side surface 40Q side towards the space between the guide members, as shown in Fig. 6.

Because the position of the absorber can be changed mutually by 180 degrees in adjacent absorbent articles, the absorbent articles can be arranged with a good balance by controlling the deviation in the thickness caused by a deviation in the absorber 4.

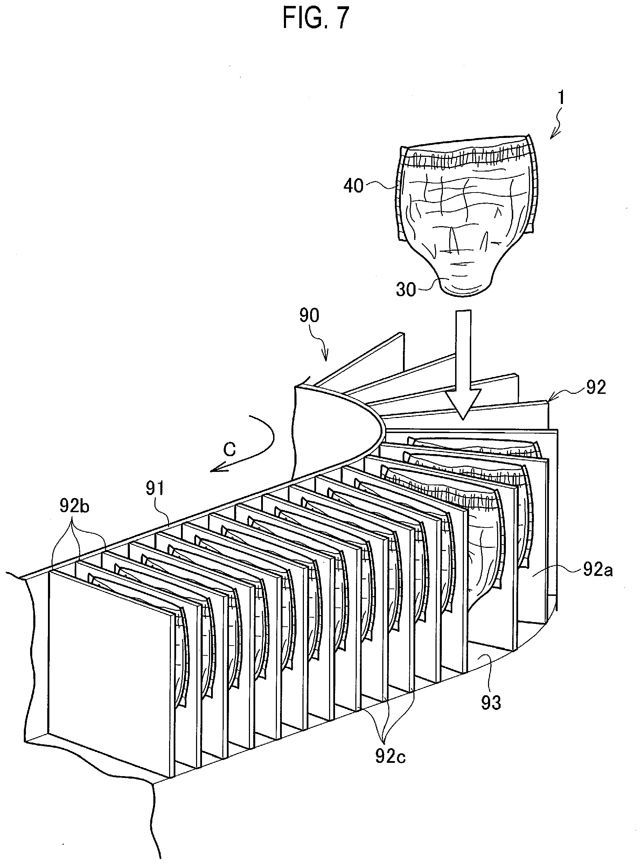

Furthermore, for example, by changing the direction with the direction-changing mechanism 80 such that the angle of the absorbent articles is rotated by 90 degrees clockwise from the state shown in Fig. 4, the conveyance mechanism 75 can be configured to feed the absorbent articles 1 from the crotch region 30 side towards the space between the guide members 92, as shown in Fig. 7.

Because the absorbent articles are in contact with the support members 93 from the crotch region side, the impact is mitigated by the absorber, and the absorbent articles can be stored in a stable state. Furthermore, as compared to other regions in which the absorber is not arranged, the crotch region 30 is relatively heavier. When the absorbent articles 1 are conveyed while being sandwiched by the guide members 92, the absorbent articles can be arranged stably as the relatively heavier crotch region 30 side is positioned at the lower side.

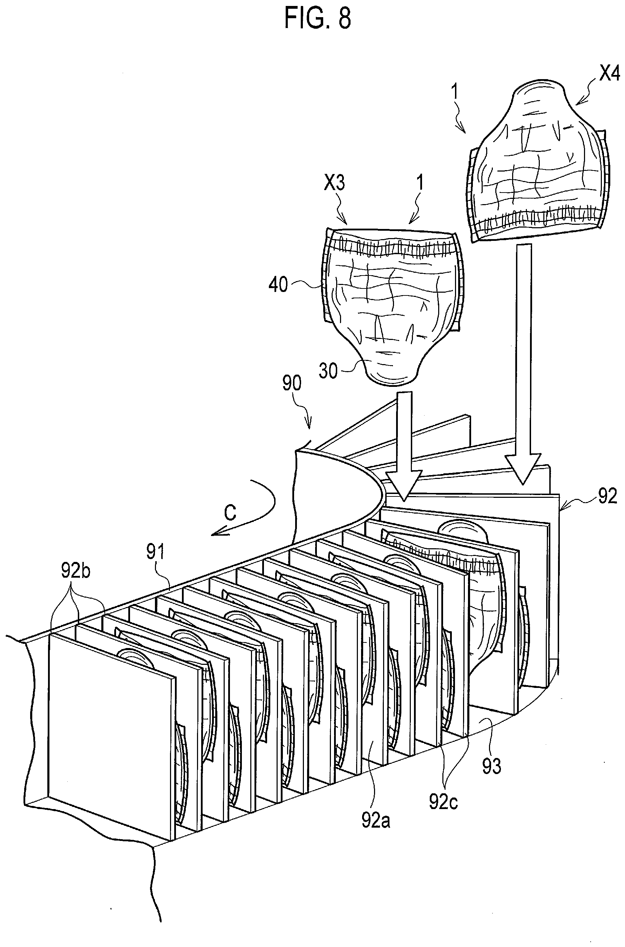

Furthermore, for example, by changing the direction with the direction-changing mechanism 80 such that the angle of the absorbent articles is in the state shown in Fig. 7, and is rotated by 180 degrees from the state shown in Fig. 7, the conveyance mechanism 75 can be configured to alternately execute a third feeding form X3 for feeding the absorbent articles 1 from the crotch region 30 side towards the space between the guide members 92, and a fourth feeding form X4 for feeding the absorbent articles from the upper surface side facing the bottom surface of the absorbent articles 1 towards the space between the guide members 92, as shown in Fig. 8.

The upper surface configuring the waistline opening region 50 of the absorbent article, and the bottom surface of the crotch region side facing the upper surface, have a relatively high rigidity in the absorbent article, so that the shape is easily stabilized. Because the absorbent articles are in contact with the support members 93 from the upper surface side or the bottom surface side, the deformation of the absorbent articles by impact can be controlled.

Additionally, as compared to other regions in which the absorber is not arranged, the crotch region 30 is relatively heavier. By feeding the absorbent articles from the bottom surface side in which the crotch region 30 is positioned towards the space between the guide members 92, the absorbent articles are arranged stably as the relatively heavier crotch region 30 side is positioned at the lower side, when the absorbent articles 1 are conveyed while being sandwiched by the guide members 92. Additionally, the waistline opening region 50 is formed in the upper surface, and it is easy to arrange the absorbent articles along the smooth surface of the support members. Therefore, by feeding the absorbent articles 1 from the upper surface side towards the space between the guide members 92, the absorbent articles can be arranged stably.

Because the position of the absorber can be changed mutually in adjacent absorbent articles, the absorbent articles can be arranged with a good balance by controlling the deviation in the thickness caused by a deviation in the absorber.

Needless to say, the present invention includes various embodiments not described herein. Therefore, the technical range of the present invention is to be defined only by the inventive specific matter according to the adequate claims from the above description.

For example, in the present embodiment, a device for manufacturing a disposable diaper as an absorbent article is explained, however, the device for manufacturing an absorbent article according to the present invention can also be applied to a device for manufacturing other absorbent articles such as a sanitary napkin and an absorbent pad.

Additionally, when feeding the absorbent articles towards the space between the guide members, the absorbent articles may be fed from the upper surface side of the absorbent articles towards the space between the guide members.

### Industrial Applicability

As it is described above, according to the present invention, the device for manufacturing an absorbent article, which has the counting mechanism for absorbent articles by storing the absorbent articles in a stable state between guide members, can be provided.

### Reference Signs List

1: absorbent article
2: topsheet
3: backsheet
4: absorber
5: waist gathers
5a: anterior waist gathers
5b: posterior waist gathers
6: leg gathers
6a: anterior leg gathers
6b: posterior leg gathers
7: web
7A: first web
7B: second web
7C: third web
10: front waistline region
10A: side edge
20: back waistline region
20A: side edge
30: crotch region
40: joining unit
40A: predetermined region
50: waistline opening region
60: leg-hole opening region
65: forming mechanism
70: cutting device
71: cutter roller
72: anvil roller
73: cutting blade
75: conveyance mechanism
76: rollers
77: endless belt
80: direction-changing mechanism
81: rotary drum
90: counting mechanism
91: endless belt
92: guide members
92a: opposing surface
92b: connecting side surface (connecting unit)
92c: tip side surface (tip)
93: support members
100, 100A: device for manufacturing an absorbent article
CD: crossing direction
CL: center line
F: horizontal direction
G: vertical direction
M1: first movement path
M2: second movement path
MD: machine direction
SL: virtual line

## Claims

1. A device for manufacturing an absorbent article (1) comprising: a forming mechanism (65) of forming a plurality of absorbent articles including a front waistline region (10), a back waistline region (20), and a crotch region (30) positioned between the front waistline region (10) and the back waistline region (20), by arranging an absorber (4) on a web (7) in which the components configuring the absorbent articles (1) are in continuity, followed by folding the web (7) along a conveyance direction, and then cutting the web (7) along the side surfaces in a widthwise direction of the absorbent articles (1) positioned at a side of the front waistline region (10) and at a side of the back waistline region (20);
a conveyance mechanism (75) of continuously conveying the absorbent articles (1) formed by the forming mechanism (65); and
a counting mechanism of counting the absorbent articles supplied in continuation from the conveyance mechanism (75), wherein
the counting mechanism includes a plurality of guide members (92) configured to retain the absorbent articles (1), a movement member (91) configured to move the guide members (92) in a direction along a horizontal direction, and support members (93) arranged on a lower side of the guide members (92) in a vertical direction, and is configured to move the absorbent articles (1) supplied from the conveyance mechanism (75) while being sandwiched by the guide members (92), and then count the absorbent articles (1),
the guide members (92) protrude out from the outer circumference surface of the movement member (91) along the horizontal direction, and are arranged adjacent to each other along the horizontal direction, and
opposing surfaces opposing each other in the guide members (92) are the surfaces that sandwich the absorbent articles (1), and are arranged along the vertical direction, **characterized in that**
the conveyance mechanism (75) is configured to feed the absorbent articles (1) along the opposing surfaces, from the upper side of the guide members (92) in the vertical direction towards the space between the guide members (92), and
the support members (93) are configured from a sponge or a rubber, or a sponge or a rubber is provided on an upper surface of the support members (93).

2. The device for manufacturing an absorbent article according to claim 1, wherein the conveyance mechanism (75) is configured to feed the absorbent articles (1) from the side-surfaces side in the widthwise direction towards the space between the guide members (92).

3. The device for manufacturing an absorbent article according to claim 1 or 2, further comprising a direction-changing mechanism (80) configured to change a direction of the absorbent articles (1) formed by the forming mechanism (65), wherein
the direction-changing mechanism (80) changes the direction of adjacent absorbent articles to mutually different directions, and
the conveyance mechanism (75) is configured to alternately execute a first feeding form for feeding the absorbent articles (1) from a first side-surface side towards the space between the guide members (92), the first side-surface being one of the two side surfaces of the absorbent articles (1), and a second feeding form for feeding the absorbent articles (1) from a second side-surface side towards the space between the guide members (92), the second side-surface side being the other side of the two side surfaces of the absorbent articles (1).

4. The device for manufacturing an absorbent article according to claim 1, further comprising a direction-changing mechanism (80) configured to change a direction of the absorbent articles formed by the cutting mechanism, wherein
the conveyance mechanism (75) is configured to feed the absorbent articles (1) from a bottom surface side in which the crotch region is positioned, or from an upper surface side opposing the bottom surface towards the space between the guide members (92).

5. The device for manufacturing an absorbent article according to claim 4, wherein the direction-changing mechanism (80) is configured to change the direction of adjacent absorbent articles to mutually different directions, and
the conveyance mechanism (80) is configured to alternately execute a third feeding form for feeding the absorbent articles (1) from the bottom surface side towards the space between the guide members (92), and a fourth feeding form for feeding the absorbent articles from the upper surface side opposing the bottom surface of the absorbent articles towards the space between the guide members (92).

## Patentansprüche

1. Eine Vorrichtung zur Herstellung eines absorbierenden Artikels (1) umfassend:
einen Ausbildungsmechanismus (65) zur Ausbildung einer Vielzahl von absorbierenden Artikeln, die einen vorderen Taillenbereich (10), einen hinteren Taillenbereich (20) und einen Schrittbereich (30), der zwischen dem vorderen Taillenbereich (10) und dem hinteren Taillenbereich (20) angeordnet ist, umfasst, mittels Anordnung eines Absorbers (4) auf einer Lage (7), in der die Komponenten, die die absorbierenden Artikel (1) ausbilden, in Kontinuität sind, gefolgt von Falten der Lage (7) entlang einer Förderrichtung und dann Schneiden der Lage (7) entlang der Seitenoberflächen in einer Breitenrichtung der absorbierenden Artikel (1), die an einer Seite des vorderen Taillenbereichs (10) und an einer Seite des hinteren Taillenbereichs (20) angeordnet sind;
einen Fördermechanismus (75) zum kontinuierlichen Fördern der absorbierenden Artikel (1), die mittels des Ausbildungsmechanismus (65) ausgebildet sind; und
einen Zählmechanismus zum Zählen der absorbierenden Artikel, die kontinuierlich von dem Fördermechanismus (75) bereitgestellt werden, wobei der Zählmechanismus eine Vielzahl von Führungselementen (92), die zum Sichern der absorbierenden Artikel (1) ausgebildet sind, ein Bewegungselement (91), das zum Bewegen der Führungselemente (92) in eine Richtung entlang einer horizontalen Richtung ausgebildet ist, und Stützelemente (93), die an einer Unterseite der Führungselemente (92) in einer vertikalen Richtung angeordnet sind, umfasst und zum Bewegen der absorbierenden Artikel (1), die von dem Fördermechanismus (75) bereitgestellt werden während sie sandwichartig von den Führungselementen (92) umgeben sind, ausgebildet ist und dann die absorbierenden Artikel (1) zählt,
die Führungselemente (92) nach außen von der äußeren Umfangsoberfläche des Bewegungselements (91) entlang der horizontalen Richtung hervorstehen und angrenzend zu einander entlang der horizontalen Richtung angeordnet sind und
gegenüberliegende Oberflächen, die einander in den Führungselementen (92) gegenüberliegen, die Oberflächen sind, die die absorbierenden Artikel (1) sandwichartig umgeben, und entlang der vertikalen Richtung angeordnet sind,
**dadurch gekennzeichnet, dass**
der Fördermechanismus (75) zum Speisen der absorbierenden Artikel (1) entlang der gegenüberliegenden Oberflächen ausgebildet ist, von der Oberseite der Führungselemente (92) in der vertikalen Richtung in Richtung des Raums zwischen den Führungselementen (92), und
die Stützelemente (93) aus einem Schwamm oder einem Gummi ausgebildet sind, oder ein Schwamm oder ein Gummi an einer oberen Oberfläche der Stützelemente (93) vorgesehen ist.

2. Die Vorrichtung zur Herstellung eines absorbierenden Artikels gemäß Anspruch 1, wobei der Fördermechanismus (75) zum Speisen der absorbierenden Artikel (1) von der Seitenoberflächenseite in der Breitenrichtung in Richtung des Raums zwischen den Führungselementen (92) ausgebildet ist.

3. Die Vorrichtung zur Herstellung eines absorbierenden Artikels gemäß Anspruch 1 oder 2, ferner umfassend einen Richtungsänderungsmechanismus (80), der zum Ändern einer Richtung der absorbierenden Artikel (1), die mittels des Ausbildungsmechanismus (65) ausgebildet sind, ausgebildet ist, wobei
der Richtungsänderungsmechanismus (75) die Richtung der angrenzenden absorbierenden Artikel zu gegenseitig unterschiedlichen Richtungen ändert, und
der Fördermechanismus (75) zum abwechselnden Ausführen einer ersten Speisungsart zum Speisen der absorbierenden Artikel (1) von einer ersten Seitenoberflächenseite in Richtung des Raums zwischen den Führungselementen (92), die erste Seitenoberfläche eine der zwei Seitenoberflächen des absorbierenden Artikels (1) ist, und eine zweite Speisungsart zum Speisen der absorbierenden Artikel (1) von einer zweiten Seitenoberflächenseite in Richtung des Raums zwischen den Führungselementen (92), die zweite Seitenoberflächenseite die andere Seite der beiden Seitenoberflächen der absorbierenden Artikel (1) ist, ausgebildet ist.

4. Die Vorrichtung zur Herstelllung eines absorbierenden Artikels gemäß Anspruch 1, ferner umfassend einen Richtungsänderungsmechanismus (80), der zum Ändern einer Richtung der absorbierenden Artikel, die mittels des Schneidemechanismus ausgebildet werden, ausgebildet ist, wobei
der Fördermechanismus (75) zum Speisen der absorbierenden Artikel (1) von einer Bodenoberflächenseite, in der der Schrittbereich angeordnet ist, oder von einer oberen Oberflächenseite, die der Bodenoberfläche in Richtung des Raums zwischen den Führungselementen (92) gegenüberliegt, ausgebildet ist.

5. Die Vorrichtung zur Herstellung eines absorbierenden Artikels gemäß Anspruch 4, wobei der Richtungsänderungsmechanismus (80) zum Ändern der Richtung von angrenzenden absorbierenden Artikeln zu gegenseitig unterschiedlichen Richtungen ausgebildet ist und
der Fördermechanismus (80) zum abwechselnden Ausführen einer dritten Speisungsart zum Speisen der absorbierenden Artikel (1) von der Bodenoberflächenseite in Richtung des Raums zwischen den Führungselementen (92) ausgebildet ist und einer vierten Speisungsart zum Speisen der absorbierenden Artikel von der oberen Oberflächenseite, die der Bodenoberfläche der absorbierenden Artikel in Richtung des Raums zwischen den Führungselementen (92) gegenüberliegt, ausgebildet ist.

## Revendications

1. Dispositif de fabrication d'un article absorbant (1) comprenant : un mécanisme de formage (65) permettant de former une pluralité d'articles absorbants comprenant une région avant de la taille (10), une région arrière de la taille (20), et une région d'entrejambe (30) positionnée entre la région avant de la taille (10) et la région arrière de la taille (20), en disposant un absorbeur (4) sur une bande (7) dans laquelle les constituants composant les articles absorbants (1) se trouvent en continuité, et puis en pliant la bande (7) le long d'une direction de transport, et en coupant ensuite la bande (7) le long des surfaces latérales selon une direction de la largeur des articles absorbants (1) positionnés au niveau d'un côté de la région avant de la taille (10) et d'un côté de la région arrière de la taille (20) ;
un mécanisme de transport (75) permettant de transporter en continu les articles absorbants (1) formés par le mécanisme de formage (65) ; et
un mécanisme de comptage permettant de compter les articles absorbants fournis dans le prolongement du mécanisme de transport (75), dans lequel le mécanisme de comptage comprend une pluralité d'éléments de guidage (92) conçus pour retenir les articles absorbants (1), un élément de mouvement (91) conçu pour déplacer les éléments de guidage (92) dans une direction le long d'une direction horizontale, et des éléments de support (93) disposés sur un côté inférieur des éléments de guidage (92) selon une direction verticale, et est conçu pour déplacer les articles absorbants (1), fournis en provenance du mécanisme de transport (75), tout étant pris en sandwich par les éléments de guidage (92), et ensuite compter les articles absorbants (1),
les éléments de guidage (92) font saillie de la surface circonférentielle externe de l'élément de mouvement (91) le long de la direction horizontale, et sont disposés adjacents les uns aux autres le long de la direction horizontale, et
les surfaces opposées qui se font face dans les éléments de guidage (92) sont les surfaces qui prennent en sandwich les articles absorbants (1), et sont disposées le long de la direction verticale, **caractérisé en ce que**
le mécanisme de transport (75) est conçu pour acheminer les articles absorbants (1) le long des surfaces opposées, du côté supérieur des éléments de guidage (92) selon la direction verticale vers l'espace entre les éléments de guidage (92), et
les éléments de support (93) sont conçus à partir d'une éponge ou d'un caoutchouc, ou une éponge ou un caoutchouc sont disposés sur une surface supérieure des éléments de support (93).

2. Dispositif de fabrication d'un article absorbant selon la revendication 1, dans lequel le mécanisme de transport (75) est conçu pour acheminer les articles absorbants (1) du côté des surfaces latérales selon la direction de la largeur vers l'espace entre les éléments de guidage (92).

3. Dispositif de fabrication d'un article absorbant selon la revendication 1 ou 2, comprenant en outre un mécanisme de changement de direction (80) conçu pour changer une direction des articles absorbants (1) formés par le mécanisme de formage (65), dans lequel
le mécanisme de changement de direction (80) change la direction d'articles absorbants adjacents en des directions différentes l'une de l'autre, et
le mécanisme de transport (75) est conçu pour exécuter alternativement une première forme d'acheminement permettant d'acheminer les articles absorbants (1) d'un côté d'une première surface latérale vers l'espace entre les éléments de guidage (92), la première surface latérale étant l'une des deux surfaces latérales des articles absorbants (1), et une deuxième forme d'acheminement permettant d'acheminer les articles absorbants (1) d'un côté d'une seconde surface latérale vers l'espace entre les éléments de guidage (92), le côté de la seconde surface latérale étant l'autre côté des deux surfaces latérales des articles absorbants (1).

4. Dispositif de fabrication d'un article absorbant selon la revendication 1, comprenant en outre un mécanisme de changement de direction (80) conçu pour changer une direction des articles absorbants formés par le mécanisme de découpe, dans lequel
le mécanisme de transport (75) est conçu pour acheminer les articles absorbants (1) d'un côté de surface inférieure dans laquelle est positionnée la région d'entrejambe, ou d'un côté de surface supérieure faisant face à la surface inférieure, vers l'espace entre les éléments de guidage (92).

5. Dispositif de fabrication d'un article absorbant selon la revendication 4, dans lequel le mécanisme de changement de direction (80) est conçu pour changer la direction d'articles absorbants adjacents en des directions différentes l'une de l'autre, et
le mécanisme de transport (80) est conçu pour exécuter alternativement une troisième forme d'acheminement permettant d'acheminer les articles absorbants (1) du côté de la surface inférieure vers l'espace entre les éléments de guidage (92), et une quatrième forme d'acheminement permettant d'acheminer les articles absorbants du côté de la surface supérieure faisant face à la surface inférieure des articles absorbants vers l'espace entre les éléments de guidage (92).
